# EUROPEAN PATENT APPLICATION

(11) **EP 0 861 903 A1**
(43) Date of publication of application: **02.09.1998**
(21) Application number: 97102951.7
(22) Date of filing: 22.02.1997
(51) Int. Cl.: C12P 19/04, C12P 19/26, C08B 37/08, A61K 31/725, C08L 5/08, G01N 33/68, C07K 17/00

(54) **Method for reversible immobilizing oligo and/or polysaccharides**

(71) Applicant: Lansing, Manfred, 46286 Dorsten-Wulfen (DE); Uhlenküken, Jochen, 48151 Münster (DE); Schmidt, Gerd, 45657 Recklinghausen (DE)
(72) Inventor: The designation of the inventor has not yet been filed
(74) Representative: Meyers, Hans-Wilhelm

(57) **Abstract**

A method for the preparation of oligo- and/or polysaccharides, which are essentially homogenous with respect to the length of a carbohydrate chain and/or the respective molecular weight from a sample having precursors of the oligo- and/or polysaccharide to be prepared, comprising oligo- and/or polysaccharides which are heterogenous with respect to the length of the carbohydrate chain and/or their respective molecular weight or do not have the appropriate length or weight comprising the steps of:
- immobilizing precursors of the oligo- and/or polysaccharides with a carbohydrate chain present in a sample, by reversible binding the oligo- and/or polysaccharides on a surface having at least one domain for a specific interaction with said oligo- and/or polysaccharides, the at least one domain being capable of interacting at least with parts of the precursor of the oligo- and/or polysaccharide chain by contacting the sample with said surface and forming a complex between at least one domain and the precursor of the oligo- and/or polysaccharides,
- treating said complex with an agent for cleaving the carbohydrate chain selectively at the site of the domains,
- removing the cleaved product and release the oligo- and/or polysaccharides bound to the domain, said oligo- and/or polysaccharides having an essentially homogenous length of the carbohydrate chain or an essentially homogenous molecular weight.

## Description

The present invention pertains to a method for preparation and detection of oligo- and/or polysaccharides with a carbohydrate chain in a sample, an oligo- and/or polysaccharides obtainable by the preparation according to the invention, a pharmaceutical composition comprising the oligo- and/or polysaccharides as well as a compound having at least one domain which are capable of interacting with at least parts of the precursors of the oligo- and/or polysaccharide chain.

Oligo- and polysaccharides are chains composed of saccharide units. A recent review, which can not be comprehensive, because of the fast expanding knowledge, has been written by A. Varki (Varki, A. (1993) Biological roles of oligosaccharides: all the theories are correct. Glycobiology, 3: 97 - 130).

Because of the fundamental role of oligosaccharides in pathological processes the scope of recent research is to develop carbohydrate based diagnostics, drugs, and biomaterials. Therefore, there is a great demand to develop a technology, which permits to produce economically oligosaccharides and/or polysaccharides with well characterized molecular weights and structures.

Other biological polymer families, like peptides and proteins, oligonucleotides and polynucleotides are well studied and the synthesis and isolation of oligonucleotides and polypeptides are well developed.

Oligo- and polysaccharides are the least well studied class of biological macromolecules. Numerous classical techniques for the synthesis of carbohydrates have been developed, but these techniques suffer the difficulty of requiring selective protection and deprotection. Organic synthesis of oligosaccharides is further hampered by the lability of many glycosidic bounds, difficulties in achieving regioselective sugar coupling and generally low synthetic yields. These difficulties, together with the difficulties of isolating and purifying carbohydrates has made this area of chemistry a most demanding one.

The use of enzymes as catalysts for the synthesis of carbohydrates has been demonstrated. Until recently, the major limiting factor to the use of enzymes in carbohydrate synthesis was the limited availability of the broad range of enzymes required to accomplish carbohydrate synthesis. Developments in genetic engineering of enzymes will make enzymes more available. At present a number of glycosyltransferases and other enzymes needed in carbohydrate synthesis are available, but are still expensive (Thiem, J. (1995) Application of Enzymes in synthetic carbohydrate chemistry. FEMS Microbiology Reviews. 16: 193 - 211; Wong C.-H. (1992) Engineering enzymes for chemoenzymatic synthesis. 10: 337 - 341)

Many techniques for the isolation of oligosaccharides are focussed on the isolation of oligosaccharides with complex, branched molecular structures. Carbohydrate binding proteins, e.g. immobilized lectins, which recognize specific sugar sequences located on glycoproteins, are used to isolate glycoproteins (Smith D.F. (1981) Glycolipids of cell surfaces: Isolation of specific sugar sequences using carbohydrate-binding proteins. Archives of Biological , Smith, D. F. (1982) Isolation of specific sugar sequences using carbohydrate-binding proteins. Methods in Enzymology, 83: 241 - 248). On the other hand immobilized oligo- and polysaccharides have been used to isolate the corresponding binding proteins.

Linear oligosaccharides are currently isolated in a semi-preparative scale by degrading large polysaccharides by ultra sonic treatment, enzymatic digestion or by other means. The mixture of oligo- and remaining polysaccharides is then loaded on a chromatographic gel permeation column and fractions containing the oligosaccharides are collected. The drawback of this method is that only classes of oligosaccharides with polydisperse molecular weights can be isolated according the characteristics of the used chromatographic media. A further drawback is that larger oligosaccharides, in general larger than 20 disaccharide units, can not be resolved using gel permeation chromatographic media. Laboratory methods exist to analyze larger oligosaccharides by different techniques (Jackson, P. (1994) High resolution polyacrylamide gel electrophoresis of fluorophore-labeled reducing saccharides. Methods in Enzymology, 230: 251 - 265). Another method for the preparation of oligo- and polysaccharides is the enzymatic synthesis of the polysaccharide using the corresponding polysaccharide synthase and the activated sugars. It was demonstrated that hyaluronan could be synthesized using the streptococcal hyaluronan synthase and UDP-N-acetyl- glucosamine and UDP-glucuronic acid including the regeneration of the UDP-sugars (Lansing, M., Prehm, P., O Regan, M., Martini, I. 1994) Italien Patent Application, Nr PD94000042; De Luca, C., Lansing, M., Martini, I., Crescenzi, F., Shen, G.-J., O Regan, M., Wong, C.-H.(1995) Enzymatic synthesis of hyaluronic acid with regeneration of sugar nucleotides. Journal of the American Chemical Society, 117: 5869 - 5870).

Accordingly, in light of their potential uses and their difficulty or the impossibility to obtain oligosaccharides with well characterized molecular weights, there exists a need for a general isolation method for the production of oligosaccharides in an efficient, cost effective, and generally applicable manner.

Because of their pathophysiological role, oligo- and/or polysaccharides are an important diagnostic tool in order to detect disorders. Furthermore, they are therapeutically useable substances of high therapeutic value.

It is an object of the present invention to provide oligosaccharides and polysaccharides with well defined molecular weights, and saccharide compositions which comprise oligo- and/or polysaccharides.

It is another object of this invention to provide a wide variety of saccharide compositions, including those not found in nature.

It is still another object to provide a process for obtaining an affinity chromatographic media useful in isolating and preparing oligo- and polysaccharides.

These and other objects are achieved by the present invention, which provides in principle an affinity chromatographic technique for preparing oligosaccharides, polysaccharides and other saccharide compositions.

According to the invention, the objects are attained by a method for the preparation of oligo- and/or polysaccharides providing substances having essentially homogenous length of their carbohydrate chain and/or their respective molecular weight. These oligo- and/or polysaccharides, also addressed as carbohydrate polymers, can easily be derived from samples in which precursors are present. Such precursors are more or less heterogenous with respect to the length of the carbohydrate chain or their respective molecular weight or do not have the appropriate chain length. The method according to the invention comprises the steps of: immobilizing precursors of the oligo- and/or polysaccharides on a surface having at least one domain for specific interaction with at least parts of the precursors of the oligo- and/or polysaccharides in the sample. The sample is contacted with said surface and forms a complex between the one or more domains and the precursors of the oligo- and polysaccharides. In order to obtain the appropriate fraction of oligo- and/or polysaccharides, which are essentially homogenous with respect to chain length and/or molecular weight the complex is treated with an agent for cleaving carbohydrate chains selectively at the site of the domains. Due to binding of the oligosaccharides to the one or more domains, they remain immobilized.

The length of the oligo- and/or polysaccharides obtainable by the method of the invention depends on the extension of the one or more domains. If the carbohydrate binding domain is relatively small, then the oligo- and/or polysaccharides obtained have a rather short carbohydrate chain length. When the oligo- and/or polysaccharides with longer chain length are prepared, it is preferred to either arrange binding domains in series, which means close to each other and preferably almost equidistant or to provide the surface of the support with at least two binding domains having a defined distance from each other.

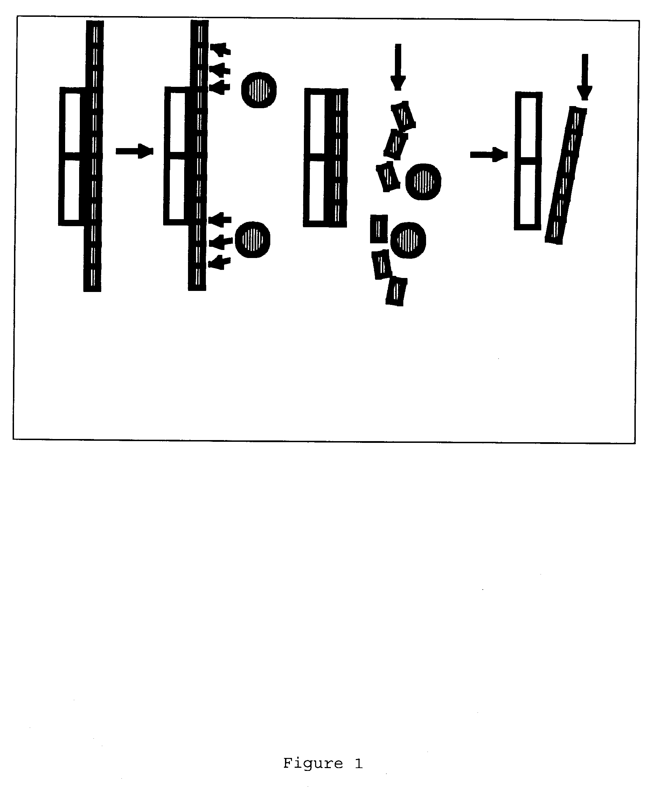

If the oligo- and/or polysaccharides to be bound are longer than the distance spanning by one or more domains, at least at one end they are extending the area of the domains forming extensions. The agent for cleaving the carbohydrate chains selectively cuts such extensions, so that a uniform chain length, defined by the distance spanning by one or more domains is obtained. Not bound material is washed away with an appropriate washing agent. After treatment with an appropriate agent the oligo- and/or polysaccharides bound by one or more domains having an essentially homogenous length of carbohydrate chain or essentially homogeneous molecular weight removed from the domains in particular by elution. The removal can take place by various conditions, for example by changing the pH or the ionic strength of the medium. See also figure 1.

Figure 1 is a schematic presentation of the purification of oligosaccharides. 1) Two carbohydrate binding domains (CBD) are immobilized onto a solid support binding a polysaccharide chain; 2) the bound polysaccharide is treated with an enzyme, which cleaves the polysaccharide at the ends of the CBD while the bound polysaccharide is protected by the CBD against the cleaving activity of the enzyme; 3) unbound material, saccharides and the enzyme are washed away and 4) finally, the oligosaccharide can be eluted.

The not bound material washed away can be brought into contact with another surface having a shorter domain for specific interaction with at least parts of the non-bound material. Again, if present extensions are removed by treatment with an agent for cleaving the carbohydrate chains selectively and after washing and eluting with appropriate agents, bound oligo- and/or polysaccharide having an essentially homogeneous molecular weight are eluted. By repeating binding, trimming, washing and elution using surfaces having step by step shorter domains it is possible to isolate a set of carbohydrate chains with stepped chain length e.g. of gradually lower molecular weight.

The process involves, in another aspect, the preparation of an affinity chromatographic medium, which consists of at least one, in particular at least two types of carbohydrate binding unit(s) arranged in series and immobilized to a solid support or to provide the surface of the support with a plurality of at least one type of binding domain having a defined distance from another binding domain.

From a sample having poly- and/or oligosaccharides, the poly- and/or oligosaccharides can be isolated by means of the affinity chromatographic medium. To achieve this affinity chromatographic medium is brought into contact with the mixture containing oligo- and/or polysaccharides or oligo- and polysaccharide compositions. Under appropriate conditions the affinity chromatographic medium binds oligo- and/or polysaccharides with a molecular weight corresponding to the number of carbohydrate binding units linked in series. After binding and washing the affinity chromatographic medium under appropriate conditions oligosaccharides with a defined molecular weight can be isolated.

According to the present invention, the term "domain" means a structural element preferably constructed by a peptide or protein which can bind a certain arrangement of saccharides particular in a linear oligo- and/or polysaccharide chain having a defined extension. The structural elements may be separated by spacers which can be a protein chain as well having no affinity to the carbohydrate or can be another polymeric structure. A domain may represent one or plurality of a carbohydrate binding site(s) of one specific kind. If the domain is only one kind of a carbohydrate binding site, at least two domains must be arranged together. Preferably, the domains may be parts of carbohydrate binding proteins, which bind to oligo- or polysaccharides. These domains bind to a defined number (m) of sugars. There are carbohydrate binding domains (CBD) which bind to hexa- (m=6), hepta- (m=7), octa- (m=8) etc. saccharides. By connecting binding domains in series (CBD)ₙ or to provide the surface of the support with a plurality of at least one binding domain having a defined distance from another it is possible to bind saccharides with n x m sugars. By choosing appropriate reaction and process conditions, it is possible to isolate oligo- and/or polysaccharides with n x m saccharide units.

Saccharide compositions with a well defined number of sugar units, prepared according with the invention find wide utility in diagnostics, therapeutics, and pharmacological applications.

In particular, the carbohydrate binding units are produced as recombinant proteins.

Preferably, the surface comprising one or more domains is a solid support. In particular, the support may be essentially constructed from an inorganic or organic polymer matrix having covalently, ionically or physically bound said one or more domains, which are preferably linked via a spacer. The chemical structure of a spacer is well known to the skilled person. The spacer serves for keeping the domain in a certain distance from the support. Particularly, the spacer is a protein having no affinity to the carbohydrate or can be another polymeric structure, as well.

The agent for cleaving the carbohydrate chain selectively is an enzyme such as an endo- or exoglycosidase or the like.

Relevant samples to be treated according the method of the invention are biological samples containing hyaluronic acid, heparin, heparan, chitin, chitosan as well as polymannuronic acid.

Subject matter of the present invention is also an oligo- and/or a polysaccharide obtainable by the method outlined above. the oligo- and/or a polysaccharide according to the invention are preferably derived from hyaluronic acid and comprising a terminal N-Ac-glucosamine-moiety or glucuronic acid-moiety. Such carbohydrate chain having an essentially defined and essentially homogenous carbohydrate chain length or molecular weight are of significance since their physiological effectivity is related to their length. Different fractions of hyaluronic acid being different in their chain length are interacting with different receptors. This leads to a different physiological response involved with the respective receptor upon binding of a certain oligosaccharide (Montesano, R., Kumar, S., Orci, L., Pepper, M.S. (1996) Synergistic effect of hyaluronan oligosaccharides and vascular endothelial growth factor on angiogenesis in vitro. Laboratory Investigation, 75, 249 - 262; Ziegler, J. (1996) Hyaluronan seeps into cancer treatment trials. Journal of the National Cancer Institute, 88, 97 - 99.).

Polysaccharides, and in particular sulfated low molecular weight polysaccharides have been found to inhibit the replication of some enveloped viruses, e.g. human immunodeficiency virus (HIV), herpes virus (HSV) or cytomegalovirus (CMV). During research on these saccharides it has been shown that the polysaccharide preparations, obtained by the methods available, are not homogenous with respect to their molecular weight. To study the interaction with the proteins of the virus there is a demand for oligo- and polysaccharides with defined molecular weight. (De Clercq, E. (1992) Anti HIV activity of sulfated polysaccharides. In: Carbo- hydrates and Carbohydrate Polymers. Yalpani, M (ed) ATL press, Mt Prospect, Il, USA, 87 - 101.)

For example, oligo- and/or polysaccharides are used as vehicles in drug delivery systems. Liposomes coated with polysaccharides are water soluble and stable against phospholipases. In addition, the chain length of the polysaccharide controls the characteristics of liposomes, e.g. half-life of a liposome and the release of the drug from the liposome (Kawaguchi, Y., Matsukawa, K., Gama, Y., Ishigami, Y. (1992) The effect of polysaccharide chain-length in coating liposomes with partial palmitoyl hyaluronates, Carbohydrate Research, 18, 139 - 142).

Therefore, the oligo- and/or polysaccharides obtainable by the present invention are useful tools in form of pharmaceutical compositions. The pharmaceutical composition may contain besides the oligo and/or polysaccharides of the present invention inert carriers or adjuvents or they can be formulated together with other pharmacologically effective substances in order to support the respective effects.

The kind of administration depends on the type of disease or disorder to be cured. Principally, the compositions of the invention can be applied in any particular manner known in the pharmaceutical art. Depending on the type of administration, special formulations or galenic preparations are to be preferred. Those are well known to the skilled person.

Subject of the present invention are also compounds having one or more domains which are capable of interacting with at least parts of precursors of the oligo- and/or polysaccharide chain. The compound of the invention comprises a peptide or protein with affinity to said oligo- and/or polysaccharide chain. The definition of the term "domain" has already been given herein above.

The method for detection of oligo- and/or polysaccharides in a sample also employs the basic method of the present invention. For the detection of a specific oligo- and/or polysaccharide said oligo- and/or polysaccharide is immobilized on a solid support. The compound with at least two domains for the specific interaction with the said oligo- and/or polysaccharide conjugated with a detection system (for example alkaline phosphatase, biotin or any other detection system) is brought into contact with said immobilized oligo- and/ or polysaccharide. If there is a sample in which the said oligo- and/or polysaccharide should be detected, or the concentration should be determined, the sample is brought in contact with the said compound conjugated to a detection system. After competing with the immobilized oligo- and or polysaccharide for the specific interaction with the compound conjugated to a detection system the unbound compound together with the oligo- and/or polysaccharide are removed. With the remaining compound still bound to the immobilized oligo- and/or polysaccharide the detection reaction is performed, which indicates the presence of the polysaccharide in the sample. By comparing the reaction of the sample with an unknown concentration of said oligo- and/or polysaccharide with the reaction of samples with known concentration of said ligo- and/or polysaccharide the amount of the oligo- and/or polysaccharide in the sample with an unknown concentration can be determined.

In a preferred embodiment, the carbohydrate binding units are immobilized by attachment to a solid support and the oligo- and/or polysaccharides to be contacted therewith are added thereto.

Preferred procedures for immobilization of carbohydrate binding units include immobilization of the carbohydrate binding units amino groups onto solid support oxirane groups or onto cyanogen bromide activated "Sepharose". Other immobilization procedures may be used, such as those described by Scouten (Scouten, W.H. (1987) A survey of enzyme coupling techniques. Methods in Enzymology, 135, 30 - 65) or others).

It will be appreciated that impairment of the binding site of the carbohydrate binding unit (domain) due to immobilization should be avoided. During the immobilization process the carbohydrate binding unit (domain) may be protected by the carbohydrate which binds carbohydrate binding unit (domain). For example, hyaluronan carbohydrate binding units (domains) may be protected with hyaluronan during the immobilization. In this way, contaminating proteins, if present, are not protected in any way during the immobilization.

The immobilized carbohydrate binding unit (domain) can be used as an affinity chromatographic medium.

The present invention is further illustrated by way of the following examples.

### A) Reagents and Molecular Cloning

DNA-Manipulations, transformation and cultivation of the appropriate host strains, expression and the isolation of the recombinant products were carried out using standard methodology as described by Sambrook, J.et al. (1989) and are known to those skilled in the art. DNA modifying reactions were performed according to the manufactors instructions.
The choice of the expression vector depends on the host strain used. Host strains are preferably microorganisms, e.g. a prokaryont or a yeast. Preferable, bacterial strains, like *Escherichia coli* strains and appropriate expression vectors, are used and are known by those skilled in the art.

### B) Isolation of Recombinant Proteins

For the production of recombinant proteins host strains are transformed with the appropriate expression vector and grown in an appropriate medium. Cells are harvested by centrifugation, resuspended in an appropriate buffer and the cells are lysed by high-pressure homogenization (French-Pressure Cell). The lysate is centrifugated to remove remaining particles.
Various methods purifying recombinant proteins which can be used in accordance with the invention have been published. The recombinant proteins may be isolated by conventional chromatographic techniques or by its binding properties to the specific saccharide. In actual practice of the invention the carbohydrate binding unit is expressed as an recombinant protein with an peptide tag which allows the purification by affinity chromatography
Homogenity and purity of the isolated protein was analyzed by SDS-PAGE (Laemmli, 1970)

### C) Affinitychromatographic Medium

In a preferred embodiment, the carbohydrate binding units are immobilized by attachment to a solid support and the oligo- and/or polysaccharides to be contacted therewith are added thereto.

### Example 1

### Cloning a hyaluronan two domain binding protein

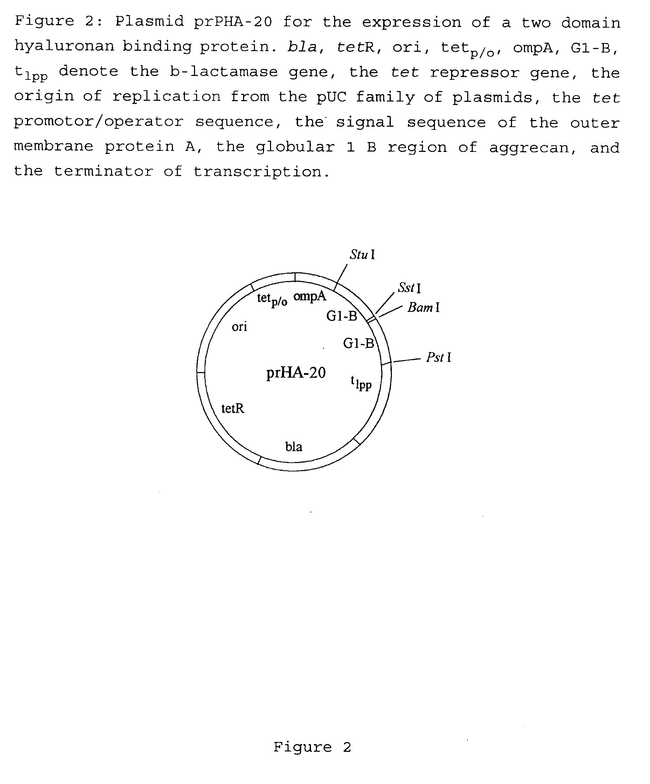

The expression vector constructed is schematically drawn in figure 2. Fragments of the gene coding for a hyaluronan binding protein (Doege, K.J., Sasaki, M., Kimura, T., Yamada, Y. (1991) Complete coding sequence and deduced primary structure of the human cartilage aggregating proteoglycan, aggrecan. Journal of Biological Chemistry, 266, 894 - 902) is precisely fused to the bacterial outer membrane protein (OmpA) signal sequence encoded by the pASK75 plasmid (Biometra GmbH, Göttingen, Germany).

The DNA-sequence coding for the G1-B hyaluronan binding domain of aggrecan was amplified by PCR using following primers (StuI-primer: 5 acagcgaggcctccctggaaagtcgtg3 , SstI- primer: 3 ccctcgagctcctcggcgaagcag5 , BamHI-primer: 5 acagc ggatccaccctggaagtcg3 , PstI- primer: 5 ctcacctgcagtctcctcggc gaag3 ). Template cDNA is prepared from RNA extracted from human cartilage. RNA is isolated according to Adams et al. (Adams, M.E., Huang, D.Q., Yao, L.Y., Sandell, L.J. (1992) Extraction and isolation of mRNA from adult articular cartilage. Analytical Biochemistry, 202, 89 - 95). cDNA synthesis was performed according the PCR Application Manual from Boehringer Mannheim (Boehringer Mannheim GmbH, Mannheim, Germany, 1995) The amplified DNA was cloned in pASK75 using the restriction sites *Stu*I, SstI and *Bam*HI and *Pst*I creating a plasmid expressing two G1-B domains separated by a small peptide spacer.
StuI-Primer 1: The PCR product amplified using the primers StuI and SstI was ligated to pASK75 digested with *Stu*I and *Sst*I resulting in the plasmid prPHA-10. The plasmid prPHA-10 is brought in *E*. *coli* JM110 by electroporation and is prepared using standard plasmid purification protocols. The second PCR product using the primers BamHI and PstI is ligated to prHA-10 digested with *Bam*HI and *Pst*I resulting in the plasmid prPHA-20, which is propagated in *E*. *coli* JM110.

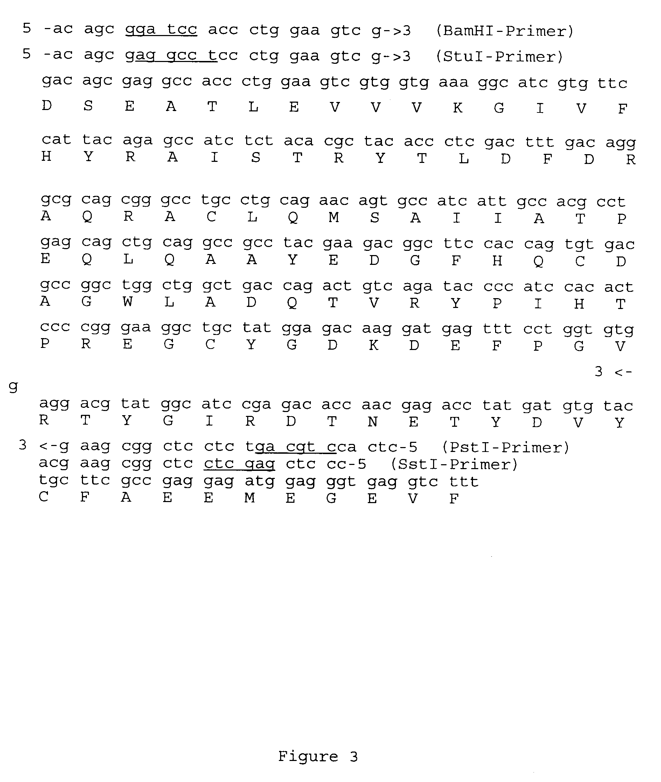

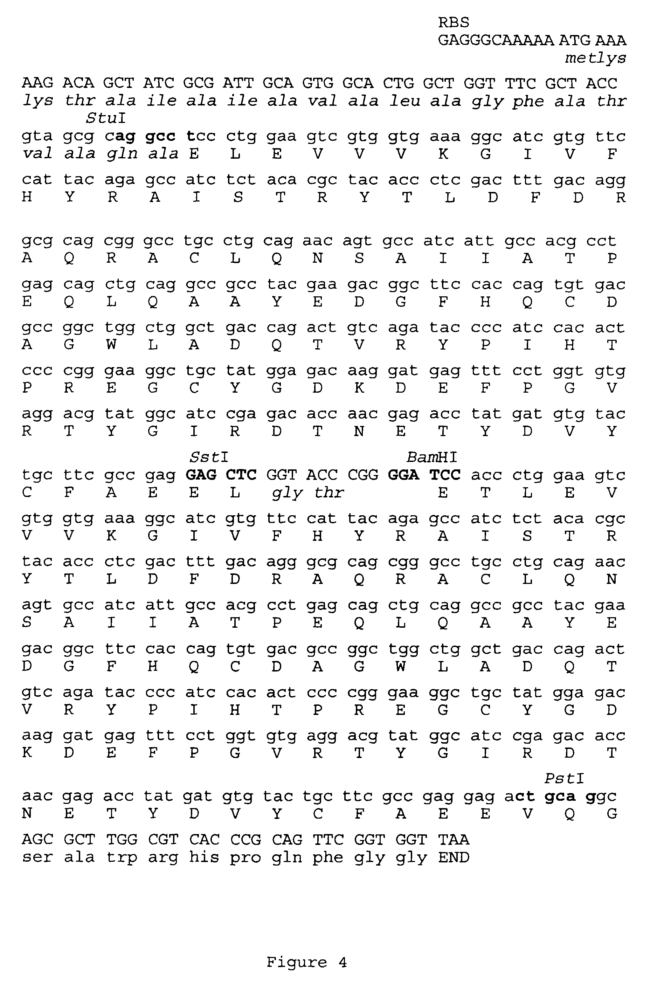

Figure 3 shows coding sequence of the G1-B domain of human aggrecan and primers for PCR amplification and cloning in pASK75 whereas figure 4 shows the coding sequence for the cloned duplicate G1-B domain starting from the RBS of the tet_{o/p} of prHA-20

### Example 2

### Isolation of a recombinant protein

A preculture is obtained by inoculating 5 ml LB medium containing 100 µg/ml ampicillin with a single colony of E. coli JM110 harbouring the plasmid prPHA-20. 250 ml LB medium containing 100 µg/ml ampicillin is inoculated with 2.5 ml of the preculture and shaken (175 rpm) at 25°C. When the culture reaches an optical density at 600 nm of 0.5 25 µl of anhydrotetrazyklin (2 mg/ml) is added and shaking is continued for 2 hours. All further manipulations are done at 4°C. *E*. *coli* JM110 cells expressing the recombinant rPHA-20 protein are harvested by centrifugation (4,500 x g, 20 min). After discarding the supernatant the cells are resuspended in 10 ml lysis buffer (100 mM Tris/HCl, 1 mM EDTA, pH 8.0) and incubated for 30 min on ice to isolate the proteins from the periplasmic space. Speroblatsa and cell debris are removed by centrifugation at 18,000 x g for 20 minutes at 4°C. After ultracentrifugation at 100,000 x g for 1 hr at 4°C the cleared supernatant is brought to 500 mM NaCl and is loaded on a column (d: 16 mm) containing 10 ml Ni²⁺NTA agarose resin (Qiagen GmbH, Heiden, Germany). The resin is washed 500 ml phosphate buffer I (20 mM NaHPO₄, 500 mM NaCl, pH 7.8) and phosphate buffer II (20 mM NaHPO₄, 500 mM NaCl, pH 6) at a flow rate of 0,5 ml/min. The recombinant rPHA-20 protein is eluted from the resin using imidazole buffer (20 mM NaHPO₄, 500 mM NaCl, 300 mM imidazole, pH 6) at a flow rate of 0,5 ml/min. Fraction containing the rPHA-20 protein were pooled together, dialyzed for 16 hrs against 5 l (20 mM NaHPO₄, pH 7.5) and concentrated by ultrafiltration using an Amicon ultrafiltration apparatus and a membrane with molecular weight cut-off of 30,000 kDalton.

### Example 3

### Preparing a affinity chromatographic matrix

The isolated rPHA20 protein (10 mg/ml) is mixed with hyaluronic acid, partially degraded by ultrasonic treatment (final concentration 0,01 mg hyaluronic acid/ml) in phosphate buffer (20 mM NaHPO₄, pH 7.5) prior to immobilization to protect the carbohydrate binding domains. Immobilization is carried out in the same phosphate buffer. The protected protein is added to dry Eupergit-C (100 mg protein/g beads) and the mixture is left to stand at room temperature with occasional mixing to ensure adequate infiltration. The extend of immobilization is monitored by loss of protein from the supernatant. A solution of protein without beads is used as control. After the immobilization is judged to be complete (90 % loss of protein from the supernatant) the beads are washed with washing buffer (20 mM NaHPO₄, 500 mM NaCl, pH 7.5) to remove unbound or loosely bound protein. Washing is continued until the absorbance at 280 nm was less than 0.1. The beads are stored at 4°C in storage buffer (20 mM NaHPO₄, 0,01 % NaN₃, pH 7.5) until required.

### Example 4

### Isolation of hyaluronan oligosaccharides with 20 sugar units

A chromatographic column (1,6 cm x 5 cm) is packed with the Eupergit-C beads to which the recombinant rPHA-20 protein recognizing 20 sugar units is immobilized (rPHA20-beads). The column is loaded with a digested preparation of hyaluronic acid (0,1 M phosphate buffer, pH 7,5; 0,25 ml/min). After washing the column with phosphate buffer to remove unbound material the column is incubated with hyaluronoglucosaminidase (E.C. 3.2.1.35) in the same buffer for 30 min at room temperature. Again the column is washed with phosphate buffer and the column is washed with a gradient from 0 to 2 M NaCl in phosphate buffer to remove unspecific bound material. Oligosaccharides are eluted from the column with 4 M guanidine chloride in phosphate buffer.

The samples are dialyzed again 0,1 M NaCl to remove guanidine chloride and are lyophilized.

### Example 5:

### Detection of Hyaluronic Acid

The wells of a CovaLink module (Nunc GmbH, Wiesbaden, Germany) is coated with hyaluronic acid according to the TechNote Vol.1, No. 9 (1995). After washing away unbound hyaluronic acid with glycine buffer (0,1 M glycine, 10 mM MgCl₂, pH 7,5) the module is briefly air dried. 50 µl of a solution containing the rPHA20 Protein (the two domain system for the specific interaction with hyaluronic acid) conjugated to alkaline phosphatase in glycine buffer is pipetted to the wells of the module. After incubation for 10 min at room temperature 50 µl of each standard solution with a known concentrations of hyaluronic acid (1 µg/ml, 0,1µgml etc) are pipetted to the wells 1B - 1 H. To the well 1A only glycine buffer is pipetted witch serves as a blank value. 50 µl of the samples with the unknown concentration of hyaluronic acid and dilution therefrom are pipetted to the wells 2A - 2H, 3A - 3H etc. After incubation for 30 min at room temperature the whole solution is removed from the wells and first the detection buffer I (0,1 M glycine, 10 mM MgCl₂, pH 10 at 37°C), and secondly 50 µl of the detection buffer II (15,2 mM p-nitrophenyl phosphate) is added to each well. After incubation for 30 min at 37°C to each well 100 µl 0.2 N NaOH is added to each well and mixed. The absorbance at 405 nm is recorded using a microtiter plate reader. By comparing the absorbance of the standards with the absorbance of the sample, the concentration of hyaluronic acid in the sample can be calculated.

## Claims

1. A method for the preparation of oligo- and/or polysaccharides, which are essentially homogenous with respect to the length of a carbohydrate chain and/or the respective molecular weight from a-sample having precursors of the oligo- and/or polysaccharide to be prepared, comprising oligo- and/or polysaccharides which are heterogenous with respect to the length of the carbohydrate chain and/or their respective molecular weight or do not have the appropriate length or weight comprising the steps of:
- immobilizing Precursors of the oligo- and/or polysaccharides with a carbohydrate chain present in a sample, by reversible binding the oligo- and/or polysaccharides on a surface having at least one domain for a specific interaction with said oligo- and/or polysaccharides, the at least one domain being capable of interacting at least with parts of the precursor of the oligo- and/or polysaccharide chain by contacting the sample with said surface and forming a complex between at least one domain and the precursor of the oligo- and/or polysaccharides,
- treating said complex with an agent for cleaving the carbohydrate chain selectively at the site of the domains,
- removing the cleaved product and release the oligo- and/or polysaccharides bound to the domain, said oligo- and/or polysaccharides having an essentially homogenous length of the carbohydrate chain or an essentially homogenous molecular weight.

2. The method according to claim 1 wherein the surface is a support comprising the at least one domain.

3. The method according to claim 2 wherein the support is an inorganic or organic polymer matrix having covalently, ionically or physically bound said at least one domain, which is preferably linked to the support via a spacer.

4. The method according to anyone of the claims 1 to 3 wherein the at least one domain is a carbohydrate binding peptide or protein having an affinity to the oligo- and/or polysaccharides to be prepared.

5. The method according to anyone of the claims 1 to 4 wherein the agent for cleaving the carbohydrate chain selectively is an enzyme such as glucosidase, hydrolase and the like.

6. The method according to anyone of the claims 1 to 5 wherein oligo- and/or polysaccharides which are heterogenous with respect to the length of the carbohydrate chain and/or molecular weight are hyaluronic acid, heparin, polymannuronic acid, chitin or chitosan containing samples.

7. An oligo- and/or a polysaccharide obtainable by the method of anyone of the claim 2 to 6.

8. The oligo- and/or polysaccharides according to claim 7 derived from hyaluronic acid containing samples comprising a terminal N-Ac-glucamin-moiety or glucuronic acid-moiety having a substantially defined and substantially homogenous carbohydrate chain length or molecular weight.

9. A pharmaceutical composition comprising an oligo- and/or a polysaccharide according to claim 7 or 8.

10. A support having a surface to which a compound is bound and which compound having at least one domain which is capable of interacting with at least parts of a precursor of the oligo- and/or polysaccharide chain comprising a peptide or protein with affinity to said oligo- and/or polysaccharide chain.

11. A method for detection of oligo- and/or polysaccharides in a sample by reversible binding the oligo- and/or polysaccharides on a surface having at least one domain for a specific interaction with said oligo- and/or polysaccharide comprising further the step of detecting the oligo- or polysaccharide from the sample bound to the surface either after elution of the bound oligo- or polysaccharide from the surface or by detecting the oligo- or polysaccharide bound on the surface.


SEQUENCE LISTING

(1) GENERAL INFORMATION:

(i) APPLICANT:
(A) NAME: Manfred Lansing
(B) STREET: Duelmenerstr. 155
(C) CITY: Dorsten-Wulfen
(E) COUNTRY: Germany
(F) POSTAL CODE (ZIP): 46286

(A) NAME: Jochen Uhlenkueken
(B) STREET: Paulstr. 22
(C) CITY: Muenster
(E) COUNTRY: Germany
(F) POSTAL CODE (ZIP): 48151

(A) NAME: Gerd Schmidt
(B) STREET: Heinrich-Hertz-Str. 13
(C) CITY: Recklinghausen
(E) COUNTRY: Germany
(F) POSTAL CODE (ZIP): 45657

(ii) TITLE OF INVENTION: Method for reversible immobilizing oligo-
and/or polysaccharides

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 348 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(iv) ANTI-SENSE: NO

(xi) SEQUENCE DESCRIPTION: SEQ ID NO: 1:

GACAGCGAGG CCACCCTGGA AGTCGTGGTG AAAGGCATCG TGTTCCATTA CAGAGCCATC 60

TCTACACGCT ACACCCTCGA CTTTGACAGG GCGCAGCGGG CCTGCCTGCA GAACAGTGCC 120

ATCATTGCCA CGCCTGAGCA GCTGCAGGCC GCCTACGAAG ACGGCTTCCA CCAGTGTGAC 180

GCCGGCTGGC TGGCTGACCA GACTGTCAGA TACCCCATCC ACACTCCCCG GGAAGGCTGC 240

TATGGAGACA AGGATGAGTT TCCTGGTGTG AGGACGTATG GCATCCGAGA CACCAACGAG 300

ACCTATGATG TGTACTGCTT CGCCGAGGAG ATGGAGGGTG AGGTCTTT 348

(2) INFORMATION FOR SEQ ID NO: 2:

(i) SEQUENCE CHARACTERISTICS:
(A) LENGTH: 116 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(iv) ANTI-SENSE: NO

(xi) SEQUENCE DESCRIPTION: SEQ ID NO: 2:

Asp Ser Glu Ala Thr Leu Glu Val Val Val Lys Gly Ile Val Phe His
1               5                   10                  15

Tyr Arg Ala Ile Ser Thr Arg Tyr Thr Leu Asp Phe Asp Arg Ala Gln
            20                  25                  30

Arg Ala Cys Leu Gln Met Ser Ala Ile Ile Ala Thr Pro Glu Gln Leu
        35                  40                  45

Gln Ala Ala Tyr Glu Asp Gly Phe His Gln Cys Asp Ala Gly Trp Leu
    50                  55                  60

Ala Asp Gln Thr Val Arg Tyr Pro Ile His Thr Pro Arg Glu Gly Cys
65                  70                  75                  80

Tyr Gly Asp Lys Asp Glu Phe Pro Gly Val Arg Thr Tyr Gly Ile Arg
                85                  90                  95

Asp Thr Asn Glu Thr Tyr Asp Val Tyr Cys Phe Ala Glu Glu Met Glu
            100                 105                 110

Gly Glu Val Phe
        115

(2) INFORMATION FOR SEQ ID NO: 3:

(i) SEQUENCE CHARACTERISTICS:
(A) LENGTH: 24 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "Primer"

(iii) HYPOTHETICAL: NO

(iv) ANTI-SENSE: NO

(xi) SEQUENCE DESCRIPTION: SEQ ID NO: 3:

ACAGCGGATC CACCCTGGAA GTCG                                        24

(2) INFORMATION FOR SEQ ID NO: 4:

(i) SEQUENCE CHARACTERISTICS:
(A) LENGTH: 24 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "Primer"

(iii) HYPOTHETICAL: NO

(iv) ANTI-SENSE: NO

(xi) SEQUENCE DESCRIPTION: SEQ ID NO: 4:

ACAGCGAGGC CTCCCTGGAA GTCG 24

(2) INFORMATION FOR SEQ ID NO: 5:

(i) SEQUENCE CHARACTERISTICS:
(A) LENGTH: 25 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "Primer"

(iii) HYPOTHETICAL: NO

(iv) ANTI-SENSE: NO

(xi) SEQUENCE DESCRIPTION: SEQ ID NO: 5:

GAAGCGGCTC CTCTGACGTC CACTC 25

(2) INFORMATION FOR SEQ ID NO: 6:

(i) SEQUENCE CHARACTERISTICS:
(A) LENGTH: 23 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "Primer"

(iii) HYPOTHETICAL: NO

(iv) ANTI-SENSE: NO

(xi) SEQUENCE DESCRIPTION: SEQ ID NO: 6:

ACGAAGCGGC TCCTCGAGCT CCC 23

(2) INFORMATION FOR SEQ ID NO: 7:

(i) SEQUENCE CHARACTERISTICS:
(A) LENGTH: 770 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(iv) ANTI-SENSE: NO

(xi) SEQUENCE DESCRIPTION: SEQ ID NO: 7:

GAGGGCAAAA AATGAAAAAG ACAGCTATCG CGATTGCAGT GGCACTGGCT GGTTTCGCTA 60

CCGTAGCGCA GGCCTCCCTG GAAGTCGTGG TGAAAGGCAT CGTGTTCCAT TACAGAGCCA 120

TCTCTACACG CTACACCCTC GACTTTGACA GGGCGCAGCG GGCCTGCCTG CAGAACAGTG 180

CCATCATTGC CACGCCTGAG CAGCTGCAGG CCGCCTACGA AGACGGCTTC CACCAGTGTG 240

ACGCCGGCTG GCTGGCTGAC CAGACTGTCA GATACCCCAT CCACACTCCC CGGGAAGGCT 300

GCTATGGAGA CAAGGATGAG TTTCCTGGTG TGAGGACGTA TGGCATCCGA GACACCAACG 360

AGACCTATGA TGTGTACTGC TTCGCCGAGG AGCTCGGTAC CCGGGGATCC ACCCTGGAAG 420

TCGTGGTGAA AGGCATCGTG TTCCATTACA GAGCCATCTC TACACGCTAC ACCCTCGACT 480

TTGACAGGGC GCAGCGGGCC TGCCTGCAGA ACAGTGCCAT CATTGCCACG CCTGAGCAGC 540

TGCAGGCCGC CTACGAAGAC GGCTTCCACC AGTGTGACGC CGGCTGGCTG GCTGACCAGA 600

CTGTCAGATA CCCCATCCAC ACTCCCCGGG AAGGCTGCTA TGGAGACAAG GATGAGTTTC 660

CTGGTGTGAG GACGTATGGC ATCCGAGACA CCAACGAGAC CTATGATGTG TACTGCTTCG 720

CCGAGGAGAC TGCAGGCAGC GCTTGGCGTC ACCCGCAGTT CGGTGGTTAA 770

(2) INFORMATION FOR SEQ ID NO: 8:

(i) SEQUENCE CHARACTERISTICS:
(A) LENGTH: 759 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(iv) ANTI-SENSE: NO

(xi) SEQUENCE DESCRIPTION: SEQ ID NO: 8:

ATGAAAAAGA CAGCTATCGC GATTGCAGTG GCACTGGCTG GTTTCGCTAC CGTAGCGCAG 60

GCCTCCCTGG AAGTCGTGGT GAAAGGCATC GTGTTCCATT ACAGAGCCAT CTCTACACGC 120

TACACCCTCG ACTTTGACAG GGCGCAGCGG GCCTGCCTGC AGAACAGTGC CATCATTGCC 180

ACGCCTGAGC AGCTGCAGGC CGCCTACGAA GACGGCTTCC ACCAGTGTGA CGCCGGCTGG 240

CTGGCTGACC AGACTGTCAG ATACCCCATC CACACTCCCC GGGAAGGCTG CTATGGAGAC 300

AAGGATGAGT TTCCTGGTGT GAGGACGTAT GGCATCCGAG ACACCAACGA GACCTATGAT 360

GTGTACTGCT TCGCCGAGGA GCTCGGTACC CGGGGATCCA CCCTGGAAGT CGTGGTGAAA 420

GGCATCGTGT TCCATTACAG AGCCATCTCT ACACGCTACA CCCTCGACTT TGACAGGGCG 480

CAGCGGGCCT GCCTGCAGAA CAGTGCCATC ATTGCCACGC CTGAGCAGCT GCAGGCCGCC 540

TACGAAGACG GCTTCCACCA GTGTGACGCC GGCTGGCTGG CTGACCAGAC TGTCAGATAC 600

CCCATCCACA CTCCCCGGGA AGGCTGCTAT GGAGACAAGG ATGAGTTTCC TGGTGTGAGG 660

ACGTATGGCA TCCGAGACAC CAACGAGACC TATGATGTGT ACTGCTTCGC CGAGGAGACT 720

GCAGGCAGCG CTTGGCGTCA CCCGCAGTTC GGTGGTTAA 759

(2) INFORMATION FOR SEQ ID NO: 9:

(i) SEQUENCE CHARACTERISTICS:
(A) LENGTH: 252 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(iv) ANTI-SENSE: NO

(xi) SEQUENCE DESCRIPTION: SEQ ID NO: 9:

Met Lys Lys Thr Ala Ile Ala Ile Ala Val Ala Leu Ala Gly Phe Ala
1               5                   10                  15

Thr Val Ala Gln Ala Glu Leu Glu Val Val Val Lys Gly Ile Val Phe
            20                  25                  30

His Tyr Arg Ala Ile Ser Thr Arg Tyr Thr Leu Asp Phe Asp Arg Ala
        35                  40                  45

Gln Arg Ala Cys Leu Gln Asn Ser Ala Ile Ile Ala Thr Pro Glu Gln
    50                  55                  60

Leu Gln Ala Ala Tyr Glu Asp Gly Phe His Gln Cys Asp Ala Gly Trp
65                  70                  75                  80

Leu Ala Asp Gln Thr Val Arg Tyr Pro Ile His Thr Pro Arg Glu Gly
                85                  90                  95

Cys Tyr Gly Asp Lys Asp Glu Phe Pro Gly Val Arg Thr Tyr Gly Ile
            100                 105                 110

Arg Asp Thr Asn Glu Thr Tyr Asp Val Tyr Cys Phe Ala Glu Glu Leu
        115                 120                 125

Gly Thr Arg Gly Glu Thr Leu Glu Val Val Val Lys Gly Ile Val Phe
    130                 135                 140

His Tyr Arg Ala Ile Ser Thr Arg Tyr Thr Leu Asp Phe Asp Arg Ala
145                 150                 155                 160

Gln Arg Ala Cys Leu Gln Asn Ser Ala Ile Ile Ala Thr Pro Glu Gln
                165                 170                 175

Leu Gln Ala Ala Tyr Glu Asp Gly Phe His Gln Cys Asp Ala Gly Trp
            180                 185                 190

Leu Ala Asp Gln Thr Val Arg Tyr Pro Ile His Thr Pro Arg Glu Gly
        195                 200                 205

Cys Tyr Gly Asp Lys Asp Glu Phe Pro Gly Val Arg Thr Tyr Gly Ile
    210                 215                 220

Arg Asp Thr Asn Glu Thr Tyr Asp Val Tyr Cys Phe Ala Glu Glu Val
225                 230                 235                 240

Gln Gly Ser Ala Trp Arg His Pro Gln Phe Gly Gly
                245                 250